# EUROPEAN PATENT APPLICATION

(11) **EP 2 319 579 A1**
(43) Date of publication of application: **11.05.2011**
(21) Application number: 08773216.0
(22) Date of filing: 18.07.2008
(51) Int. Cl.: A61N 1/44

(54) **AN APPARATUS OF LOW STRENGTH ELECTRIC FIELD NETWORK-MEDIATED DELIVERY OF DRUG TO TARGET CELL OF LIVER**

(71) Applicant: Suntek Medical Scientific And Technologies (Shanghai), Pudong New Area, Shanghai 201201 (CN)
(72) Inventor: SEN, Luyi, Shanghai 201201 (CN)
(74) Representative: Brown, David Leslie
(86) International application number: PCT/CN2008/071688
(87) International publication number: WO 2010/006483

(57) **Abstract**

A drug delivery apparatus guided by micro electrical field network for target cells of liver comprises a low-voltage micro- electrical field generator and a drug delivery catheter which gets into a liver through an endoscope or indwelling catheter along blood vessels, wherein at least two drug infusion holes and a first array of electrodes are provided at the front end of the drug delivery catheter; a second array of electrodes is set on the exterior of the liver; the first array of electrodes and the second array of electrodes are connected with the pulse output terminal of the low-voltage micro-electrical field generator; the drug delivery catheter is connected through with a drug transportation device.

## Description

### Background

### Technical Field

The present invention relates to the medical field, especially, to a medical apparatus that delivers drugs to a specific target and is helpful to the permeability of the drugs.

### Brief Description of Related Arts

Primary hepatocellular carcinoma (hereinafter "liver cancer"), also called "liver tumour", is the most common malignant primary liver tumour. Liver cancer is one of three main fatal cancers among many Asian and African countries.

According to the statistics by the Ministry of Health, about 130,000 people in my country die of liver cancer every year, accounting for 40 % of the global total death amount dying of liver cancer.

The onset of the liver cancer is usually not obvious, so once the apparent symptom appears, it has already come to the intermediate or advanced stage. Only 10 to 15% of patients may have surgeries to remove the tumour; but, even the tumour is removed, the reoccurrence still can reach as high as around 60%. Due to the non-obvious symptom of the early stage, it is not easy to notice it; so, once found out, above half of these patients have already come to the intermediate or advance stage. If without any treatment, these patients during the intermediate or advance stage usually have about 3 month of average time to survive. It can be seen that, therefore, liver cancer is the worst malignant tumour in terms of prognosis.

As a supporting and auxiliary treatment method to the surgical removal, chemotherapy is very common.

However, since the systemic chemotherapy side effect is quite strong, such as gastrointestinal reaction and bone marrow suppression and etc., patients can not stand the suffering and have to only choose to do intermittent treatment. During intermittent rest times, however, the growth of cancer cells is much higher than normal cells, thus, the curative effect is poor and the systemic chemotherapy is not helpful for increasing apparently the survival rate and the survival time.

TACE (Transcatheter Arterial Chemoembolization) is the first choice for non-operative treatments nowadays, which injects chemotherapy drugs into tumour supplying arteries so that local drug concentration will be dozens of times higher than the systemic chemotherapy and the arterial embolism, after use of the drugs, can make the tumour shrink because of lacking blood. However, this "transient" increase of the drug concentration in the liver tissue can not greatly increase the drug concentration in the tumour cells, only less than 20% of patients' tumors getting shrinked, most drugs still going to the systemic circulation, and with strong side effects.

At the same time, the arterial embolism often causes vessel occlusion, so that repetitive treatments are not allowed; in addition, it might leads to the increase of endogenous VEGF (Vascular Endothelial Growth Factor), and then the vessels in tumors proliferate largely; thus, the curative effects are not ideal.

It is well known that the overwhelming majority of anti-cancer drugs can not take effect until these drugs penetrate into cells. However, current ways of administering drugs can only allow 5-15% of drugs to penetrate into cells. Therefore, in order to reach the treatment effects, increasing the dose of drugs is needed, but it will necessarily cause local and/or systemic side effects greatly.

To enable more dosage to get into the inside of cells, the issue of increasing membrane permeability has been raised.

Electroporation is the most applied method in the past, which uses electricity to increase the membrane permeability. Electroporation is the most effective method in the current technologies that introduce exogenous gene into living cells, and is similar with or superior over viruses and other methods in terms of the effectiveness of transforming gene inside the living cells.

Specifically, electroporation is the kind of technology that adopts an electrical pulse generation device to apply electrical pulses during a short period of time and with a certain intensity into cells or tissues, and that introduces exogenous gene into issues or organs of targeted animals through action of electrical fields.

As being able of introducing exogenous gene effectively, being applied into many kinds of tissues and organs and having high efficiency, reports relating to applying in vivo electroporation method into transgenic research get increased in recent years, and the advantages of electroporation get more and more obvious; therefore, it is a very good method of introducing in vivo gene.

The basic principle of Electroporation is as follows: the electrical pulses (electrical stimulation) of the electrical pulse generation device can lead to instability of cell membrane and further forms holes (or tiny passages) with the size of Nanometer grade; these passages can sustain for from milliseconds to seconds and then restore by themselves and finally take on a specific "permeable" status. At the "permeable" status, the cell membrane allows DNA, enzyme, antibody and other macromolecules get into cells through the cell membrane. Not only makes electroporation method gene treatment possible, but also other fields, such as transdermal drug delivery and chemotherapy.

Ever since the early 1980s, Electroporation has been adopted as a research tool to introduce DNA, RNA, protein, other macromolecules, liposome, emulsion microsphere or whole virus particles into living cells.

Electroporation is often applied into in vitro gene transfection. It has been reported that a pair of needle-shaped or plate-shaped electrodes can be used in the tumors, liver and myocardium of rodents to do gene transfection; but this kind of research work is limited. In recent years, electroporation catheters are just adopted to deliver heparin to arterial walls of rabbits to increase drug delivery efficiency markedly.

A Chinese patent entitled "System and method for transdermal delivery", with publication date being 01/31/2007, with publication number being CN1905920A, can be seen as a paradigm or guidance of the published electroporation technology.

However, on the other hand, the electrical pulses with high-strength electrical field generated by current electrical pulse generation device may lead to permanent destruction for the cell membrane (cell lysis). According to current known knowledge, the voltage, which is applied to any type of cells, whole embryo or embryo's heart in sample cups, must reach 200-1500 V/cm, while when a needle-shaped or plate-shaped electrodes are used, the voltage, which is applied to any tissue inside bodies, must reach 100-200 V/cm; if we apply the electroporation into organs (such as heart) of big animals or human, the voltage must reach thousands V. But, it will cause damage of a great deal of tissues; thus, the technology has not yet been applied to the clinic.

Meantime, because the electroporation will produce a lot of heat locally and instantly, exogenous gene or DNA transfection reagent needs to be kept in a lower temperature (such as 4 ° C) during the operation, and at the same time, the applied parts of the electroporation operation need to be cooled down; therefore, these measures bring certain limitations.

The electroporation system was used to deliver dermal medications, which used 2-6 heads of needles to apply high voltage and short time pulses on skins. Due to the direct damage and the impact of the high voltage, the system caused obvious skin damages and infection, and accordingly its application was limited.

Meantime, in clinical practice, chemical medications or exogenous gene injection is applied to rats' tissues, or skins, bones or tumors in living organisms. For in vivo transgene of a whole organ, only few researches are done with direct gene injection and single-needle electrodes insertion, or six-needle electrodes insertion, to rats', mice' or cat's livers, which method can only take effect to the issues that are in the range around the needles, with diameter less than 0.8 cm; moreover, the injury resulting from the needles are big; thus, it can be used to body surface at most, not to big organs of human body, such as heart, liver, lung, kidney and etc.

Therefore, it is urgent to provide a drug delivery apparatus in the medical research and clinical treatment, which is able to improve the "permeability" of cell membranes and increase the drug amount taken by them not only, but also not to cause damages to the targeted cells or organs, and moreover to achieve the delivery effect of "low dosage and long time".

### Summary of the Present Invention

The object of the present invention is to provide a drug delivery apparatus guided by micro electrical field network for target cells of liver, which, by way of a multi-dimension (or three dimensional) low (or micro) voltage electrical field, is able to improve apparently the "permeability" of cell membrane and increase greatly the drug amount taken by target cells or organs not only, but also not to cause damages to the target cells or organs, and further to enhance the targeting aiming at liver cancel cells at the most extent and reduce the systemic side effects for patients drastically

According to the present invention, the drug delivery apparatus guided by micro electrical field network for target cell of liver comprises a low-voltage micro-electrical field generator which could provide a programmable micro electrical field network, and a drug delivery catheter which gets into the liver through an endoscope or indwelling catheter along blood vessels, wherein at least two drug infusion holes and a first array of electrodes are provided at the front end of the drug delivery catheter and a second array of electrodes is set on the exterior of the liver or on the body surface corresponding to the liver; the first array of electrodes and the second array of electrodes are connected with the pulse output terminal of the low-voltage micro-electrical field generator; the drug delivery catheter is connected with a drug store-transportation device.

Moreover, the distribution of the first array of electrodes is multiple loop-shaped, dot-shaped or strip-shaped electrodes.

The distribution of the second array of electrodes is multiple strip-shaped or mesh-shaped electrodes.

Specifically, the first array of electrodes and the second array of electrodes are metallic conductive wires or foils set in an insulating film layer.

The metallic conductive wires or foils set in the insulating film layer are in the shape of strips or meshes.

The metallic conductive wires or foils set in the insulating film layer and in the shape of the strips or meshes have the structure form of positive electrode and negative electrode being arranged alternately.

Moreover, the first array of electrodes and the second array of electrodes constitute a lattice-shaped electrode array on the surface of the insulating film layer through conductive dots; the conductive dots are connected with the first and second array of electrodes and penetrate the surface of the insulating film layer.

The positive and negative electrodes of the first and second array of electrodes, after gathering, are connected with the positive and negative pulse output terminal of the low-voltage micro-electrical field generator, respectively, and then form a three-dimensional, even and dense micro electrical field net on the surface of or/and in the liver.

Compared to the current technology, the present invention has the following advantages:
1. The first array of electrodes and the second array of electrodes are combined, so that a multi-dimensional micro electrical field net is established on the surface of and in the liver. The electrical field net distributing evenly and densely over the liver can make temporary structural change happen to the membrane of each and every cell, and therefore can increase the permeability, electrical activity and affinity of the membrane.
2. The low/micro electrical field retains the integrality of the membrane greatly and will not cause damages to target cells or organs, so that avoid the injury to cells.
3. When drugs are delivered to each cell of the liver through blood vessels, the combination of electrodes and the drug delivery catheter provides pulse waves to increase the permeability of the membrane of each cell of the liver and make drugs get into each cell through the membrane, and finally helps drugs get into cells.
4. The direct contact of the electrodes and the liver organ has eliminated completely the attenuation of the electrical field strength caused by the distance between the electrodes and targets cells.
5. Because drugs are delivered direct to the targeted organ, the most important is that no obvious local and systemic side effects are produced except for high efficiency of drug delivery.

### Brief Description of Drawings

Fig.1 is a schematic structure diagram of the front end of the drug delivery catheter of the present invention.
Fig.2 is a schematic structure diagram of the second array of electrodes of the present invention.
Fig.3 is a schematic structure diagram of the joint area of the electrodes and conductive dots.
Fig.4 shows the schematic structure diagram of the electrical field generated by the first array of electrodes and the second array of electrodes in the liver.
Fig.5 is a schematic structure diagram of another distribution of the first array of electrodes.
Fig.6 is a schematic structure diagram of another distribution of the second array of electrodes.
Fig.7 is an enlarged section view of C part of Fig.6.

In the drawings, 10 denotes drug delivery catheter; 11- first array of electrodes; 12- drug infusion hole; 13- wires; 20- insulating film layer; 21 and 22 denote positive and negative electrodes made of metallic conductive wires or foils respectively; 23-conductive dots; 24 and 25- bus bar; 30- liver; 31- targeted cancerous area; 32- blood vessels; 33- electromagnetic wires (electrical field wires); 34- multidimensional or three-dimensional micro electrical field net.

### Detailed Description of the Preferred Embodiments

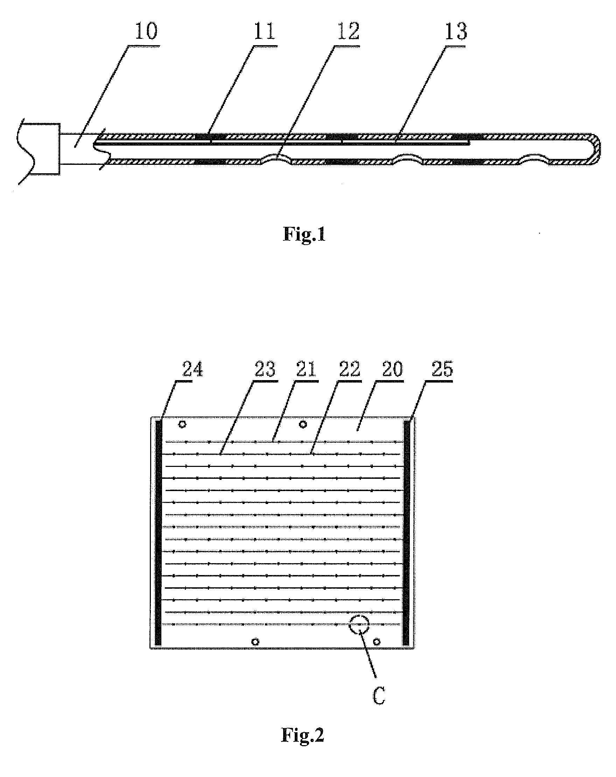

As shown in Fig.1, at least two drug infusion holes 12 and a first array of electrodes 11 are set at the front end of the drug delivery catheter 10, and wires 13 are connected among the electrodes.

The distribution of the first array of electrodes is multiple loop-shaped, dot-shaped or strip-shaped. The present figure shows the loop-shaped electrodes.

As shown in Fig.2, the second array of electrodes is metallic conductive wires or foils 21 and/or 22 set in the insulating film layer 20.

The metallic conductive wires or foils take on the shape of strips or meshes, set in the insulating film layer.

The electrodes in the present figure take the strip-shaped structure. Its positive electrode 21 and negative electrode 22 are arranged alternately, and after gathering together through the bus bars 24 and 25 located at both sides of the insulating film layer 20, are connected electrically through wires with the positive pulse output terminal and negative pulse output terminal of the low-voltage micro electrical field generator, respectively.

As seen in the drawings, the metallic conductive wires or foils 21, 22, constituting the electrode array, form a lattice-shaped electrode array on the surface of the insulating film layer through conductive dots 23; the conductive dots 23 are connected with the metallic conductive wires 21 and 22 and penetrate the surface of the insulating film layer 20.

Concerning the relevant materials about the low-voltage micro-electrical field generator, please refer to Applicant's Chinese patent 200810036767.4 or 20082005784.2, and an International patent application PCT/CN2008/001022. The relevant contents are not going to be described herein.

It needs to be stated that the published contents of above-mentioned references should not be regarded as the limitation to the present invention, but the background or explanatory information.

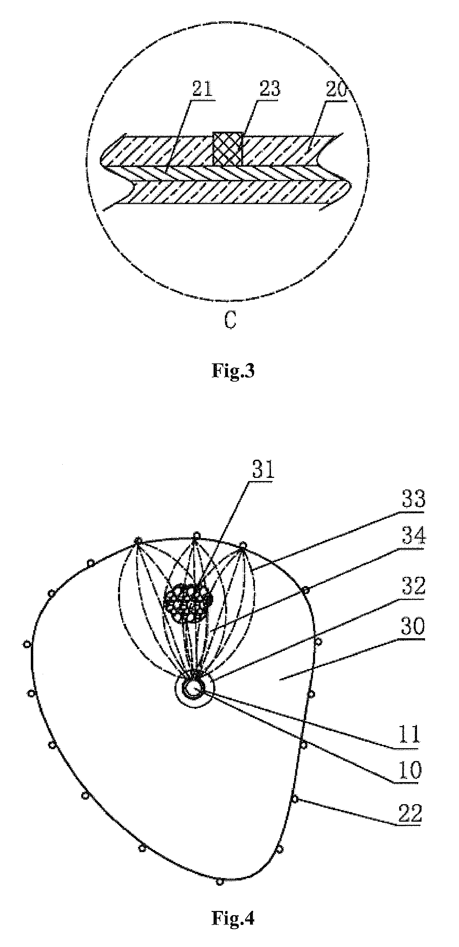

In Fig.3, the metallic conductive wires or foils (e.g.: the positive electrode 21 in the figure) form a lattice-shaped electrode array on the surface of the insulating film layer through conductive dots 23; the conductive dots are connected with the metallic conductive wires 21 and 22 and penetrate the surface of the insulating film layer 20.

In practical making, platinum or tungsten gold can be used as the conductive dots; it is also viable to get the conductive dots by chemical plating conductive metals (such as copper, silver etc.) on the penetrating points. Since this is the current technology, so it is not going to be described herein.

In Fig.4, the first array of electrodes 10 is provided on the drug delivery catheter 10 (it is the positive electrode herein) which gets into the liver 30 along the blood vessel 32. The second array of electrodes 22 (it is the negative electrode herein), with the shape of strips or meshes, is provided on the exterior of the liver organ. After the positive electrode and negative electrode are connected with the low-voltage micro-electrical field generator (not drawn in the figure), under the drive and control of the generator, a multidimensional micro electrical field net 34 is established between the positive and negative electrodes. The micro-electrical field net 34 is consisting of multiple group of electromagnetic wires 33; then, the targeted cancerous area 31 is put in the action of the electrical field.

To make the drawings be neat, only partial electrical field wires are drawn. Actually, the electrical field wires exist in between each pair of the positive and negative electrode, which is common knowledge.

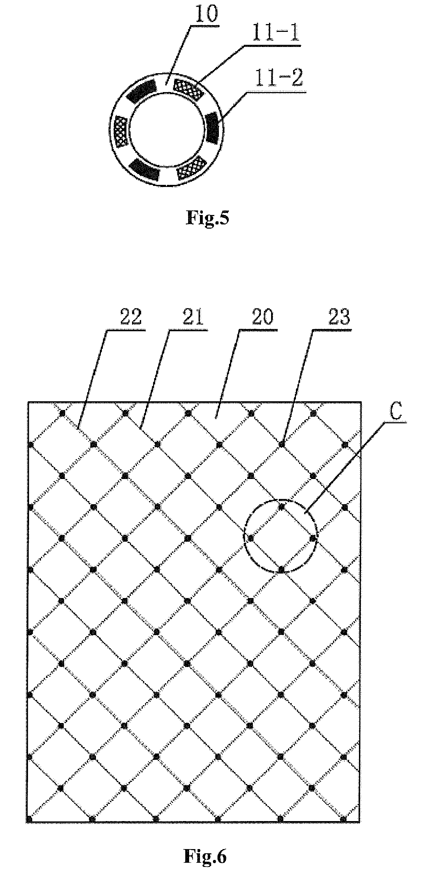

In Fig.5, another structure of the first array of electrodes, which are located at the front end of the drug delivery catheter 10, is disclosed, wherein the first array of electrodes is comprised of a positive electrode 11-1 and a negative electrode 11-2, which are distributed alternately; the first array of electrodes take on the shape of strips.

It is also viable to arrange the first array of electrodes with the shape of dots, but the details are not going to be recited herein.

Others in Fig.5 are the same as Fig.1.

As shown in Fig.6, another distribution structure of the second array of electrodes is disclosed, wherein the metallic conductive wires or foils 21 or/and 22 that are set in the insulting film layer 20 take on the shape of meshes. The metallic conductive wires or foils 21 are positive electrode and 22 are negative electrode, and form an electrode lattice on the surface of the insulting film layer after going through the conductive dots 23 respectively.

Others in Fig.6 are the same as Fig.2.

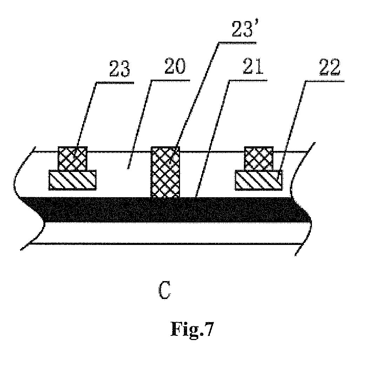

In Fig.7, the positive electrode 21 and negative electrode 22 distribute alternately in the insulating film layer 20, going through the conductive dot 23 and 23' respectively and forming an electrode lattice on the surface of the insulating film layer.

Others in Fig.7 are the same as Fig.2 and Fig.3.

It needs to be point out that the structural form of the first and second array of electrodes should not be limited to the above-described ways. To obtain better effects of electrical field distribution and/or superposition of electrical field strength, various geometric shapes (such as strips, meshes, loops, irregular curves, and even some conics) can be tried as the electrode distribution shape.

For convenient operation, as a special example, the second array of electrodes can even direct adopt metallic mesh-shaped electrodes to cover the body surface corresponding to the liver, which can take the same effect.

Because of the direct contact of electrodes and tissues, it has completely eliminated the attenuation of the electrical field strength greatly, which results from the distance existing between the electrodes and target cells, and accordingly is helpful to achieve the purpose of increasing the permeability of cell membrane.

Among the above-described technical plans and embodiments, the reason why the electrode distributions of various geometric shapes are adopted is to superpose as more electrical field strength as possible on the surface of or in the liver, and on even some targeted area inside it, and to increase as much permeability of the membrane of each cell of the liver organ as possible to help drugs get into cells through as high electrical field action as possible. Moreover, countless micro-electrical fields cross, superpose and penetrate each other in tissues, whereby high-voltage is no longer needed and the purpose of increasing the permeability of membrane can be achieved. Meanwhile, multiple consecutive pulses can be used, such as the method of alternate-cluster program pulse (Burst), to increase the working time.

After the above-said technical plan has been used, it is tested that when pulse voltage with Volt grade or mV grade is used, the method can last for 5 hours without any injury to the cells of tissues, and even allow at least 60-70% of drugs get into cells, which is far superior over the drug delivery efficiency of the current technology (using pulse voltage with hundreds Volt and even higher grade but only 5-15% of amount of drugs getting into cells) and improves the targeting aiming at liver cancer cells to the fullest extent and reduces systemic side effects of patients greatly.

One skilled in the art will understand that the embodiments of the present invention as shown in the drawings and described above is exemplary only and not intended to be limiting.

It will thus be seen that the objects of the present invention have been fully and effectively accomplished. Its embodiments have been shown and described for the purpose of illustrating the functional and structural principles of the present invention and is subject to change without departure from such principles. Therefore, this invention includes all modifications encompassed within the spirit and scope of the following claims.

The present invention can be applied widely into the clinical medication and gene treatment.

## Claims

1. A drug delivery apparatus guided by micro electrical field network for target cells of liver, comprising a low-voltage micro electrical field generator which could provide a programmable micro electrical field network and a drug delivery catheter which gets into a liver through an endoscope or indwelling catheter along blood vessels, wherein at least two drug infusion holes and a first array of electrodes are provided at the front end of the drug delivery catheter; a second array of electrodes is set on the exterior of the liver or on the body surface corresponding to the liver; the first array of electrodes and the second array of electrodes are connected with the pulse output terminal of the low-voltage micro-electrical field generator; the drug delivery catheter is connected with a drug store-transportation device.

2. The drug delivery apparatus guided by micro electrical field network for target cells of liver set forth in claim 1, wherein the distribution of the first array of electrodes is multiple loop-shaped, dot-shaped or strip-shaped electrodes.

3. The drug delivery apparatus guided by micro electrical field network for target cells of liver set forth in claim 1, wherein the distribution of the second array of electrodes is multiple strip-shaped or mesh-shaped electrodes.

4. The drug delivery apparatus guided by micro electrical field network for target cells of liver set forth in claim 1, the first array of electrodes and the second array of electrodes are metallic conductive wires or foils set in an insulating film layer.

5. The drug delivery apparatus guided by micro electrical field network for target cells of liver set forth in claim 4, wherein the metallic conductive wires or foils set in the insulating film layer take on the shape of strips or meshes.

6. The drug delivery apparatus guided by micro electrical field network for target cells of liver set forth in claim 5, wherein the metallic conductive wires or foils set in the insulating film layer are arranged in such a manner that the positive electrode and negative electrode are arranged alternately.

7. The drug delivery apparatus guided by micro electrical field network for target cells of liver set forth in claim 1, the first array of electrodes and the second array of electrodes form a lattice-shaped electrode array on the surface of an insulating film layer through conductive dots; the conductive dots are connected with the first array of electrodes and the second array of electrodes and penetrate the surface of the insulating film layer.

8. The drug delivery apparatus guided by micro electrical field network for target cells of liver set forth in claim 1, after gathering together, the positive and negative electrodes of the first and second array of electrodes are connected with the positive and negative pulse output terminal of the low-voltage micro electrical field generator, respectively, and then form a multidimensional, even and dense micro electrical field net on the surface of or (and) in the liver.
